# EUROPÄISCHE PATENTSCHRIFT

(11) **EP 1 643 940 B1**
(45) Veröffentlichungstag und Bekanntmachung des Hinweises auf die Patenterteilung: **03.12.2008**
(21) Anmeldenummer: 04739951.4
(22) Anmeldetag: 16.06.2004
(51) Int. Cl.: A61F 2/36

(54) **HÜFTPROTHESE MIT EINEM IN DEN OBERSCHENKELKNOCHEN EINZUSETZENDEN SCHAFT**
HIP PROSTHESIS PROVIDED WITH A SHAFT INSERTED INTO THE FEMUR
PROTHESE DE HANCHE COMPRENANT UNE TIGE A INSERER DANS L'OS DE LA CUISSE

(30) Priorität: 16.07.2003 EP 03016157
(43) Veröffentlichungstag der Anmeldung: 12.04.2006
(73) Patentinhaber: WALDEMAR LINK GmbH & Co. KG, 22339 Hamburg (DE)
(72) Erfinder: KELLER, Arnold, 23863 Kayhude (DE)
(74) Vertreter: Glawe, Delfs, Moll
(86) Internationale Anmeldenummer: PCT/EP2004/006485
(87) Internationale Veröffentlichungsnummer: WO 2005/007039

(56) Entgegenhaltungen:
- EP-A- 0 567 349
- EP-A- 0 780 106
- WO-A-93/08770
- DE-U- 29 522 382
- FR-A- 2 633 509
- US-A- 5 728 161
- US-A- 5 755 811
- US-B1- 6 168 632

## Beschreibung

Der dem Oberschenkel zugeordnete Teil einer Hüftprothese ist mit einem Schaft ausgestattet, der in eine Höhlung eingesetzt wird, die nach der Resektion des Hüftkopfs und Kopfhalses mittels eines geeigneten Werkzeugs im spongiösen, inneren Querschnittsbereich des Oberschenkelknochens geformt wird. Wenn der Schaft zementfrei eingesetzt wird, bemüht man sich, die Form der Höhlung möglichst weitgehend komplementär übereinstimmend mit der Form des Schafts auszubilden, damit die Prothese nach dem Einsetzen des Schafts sicher und fest sitzt. Dabei ist der proximale Schaftabschnitt, der im metaphysären Bereich des Knochens (etwa oberhalb des kleinen Trochanters) zu liegen kommt, so ausgebildet, daß er nicht nur vertikale Kräfte in Richtung des Oberschenkelknochens sondern auch quer dazu verlaufende und insbesondere nach medial gerichtete Kräfte übertragen kann. Damit auch die.nach ventral und dorsal weisenden Oberflächenteile des proximalen Prothesenschafts an der Kraftübertragung auf den Knochen beteiligt werden, ist es bekannt, ihre Oberfläche so auszubilden, daß ein formschlüssiger Verbund mit dem Knochengewebe zustandekommen kann. Dafür stehen zwei Möglichkeiten zur Verfügung, nämlich zum einen eine Mikro-Rauhigkeit der Oberfläche, die beispielsweise durch Glasstrahlen, poröse Beschichtung oder dergleichen zustande kommt und dem Knochen Gelegenheit gibt, in die Vertiefungen und Poren einzusprossen, und zum anderen Rippen, die von dem Schaftgrundkörper vorspringen. Diese beiden Möglichkeiten können auch gemeinsam angewendet werden. So ist es bekannt (EP-B-761 183; US-A-5755811) auf den dorsalen und ventralen Flächen des proximalen Schaftgrundkörpers Rippen aufzusetzen, die in Längsrichtung des Schafts verlaufen und deren Querschnitt sich von distal nach proximal keilförmig vergrößert. Derartige Rippen werden im folgenden als Keilrippen bezeichnet. Für die Herstellung der Höhlung wird eine Raspel verwendet, deren Gestalt dem Schaftgrundkörper ohne die Rippen entspricht. Beim Einsetzen des Schafts bildet der Schaftgrundkörper mit der Oberfläche der Höhlung einen Preßsitz. Die Keilrippen schneiden beim Einsetzen der Prothese in das spongiöse Knochengewebe ein. Aufgrund ihrer Keilform verdrängen und verdichten es. Dies trägt zum festen Sitz der Prothese bei. Dabei ergibt sich zum einen ein makroskopischer Formschluß zwischen dem Knochen und dem Prothesenquerschnitt dank der Rippe. Zum anderen ergibt sich nach dem Einwachsen von Knochengewebe in die rauhe oder porige Oberflächenstruktur ein mikroskopischer Formschluß. Diese beiden Wirkungen treten offensichtliche unabhängig voneinander ein und werden bei der Konstruktion einer Prothese ohne gegenseitige Rücksicht eingesetzt.

Die erwähnte Kompression des Knochenmaterials beim Einsetzen einer Keilrippe darf selbstverständlich nicht dazu führen, daß der Knochen gesprengt wird. Diese Gefahr ist um so größer, je breiter eine Rippe ist, weil sie dann der Knochenrinde eine um so größere Keilfläche zuwendet und eine um so größere Keilkraft gegen die Knochenrinde erzeugt. Bekannte Keilrippen sind deshalb schmal ausgeführt (EP-B-761 183, EP-B-159 462). Aus demselben Grunde bemüht man sich, die Keilwirkung der Rippen in die tangentiale Richtung zu lenken (DE-U-295 22 382, Blatt 4, Zeile 22). Zwar sind auch breite Rippen bei zementfrei einzusetzenden Hüftprothesen bekannt (EP-A-10 70 490; EP-A-567349). Dabei handelt es sich aber um Rippen, die zur Verdrängung und Kompression von Knochenmaterial aufgrund ihrer Form nicht geeignet sind, und deshalb eine solche Formung der Höhlung verlangen, daß ihr Volumen und ihre Form von vornherein in der Höhlung berücksichtigt wird. Bekannt sind ferner verhältnismäßig breite, von distal nach proximal sich verbreiternde Schaftvorsprünge an Prothesenschäften, die zur zementierten Implantation bestimmt sind und bei denen daher das zugehörige Werkzeug so ausgebildet ist, daß die künstliche Knochenhöhlung voluminöser ist als der Schaft einschließlich seiner rippenartigen Aufsätze.

Der Erfindung liegt die Aufgabe zugrunde, einen Prothesenschaft zur zementfreien Implantation mit Keilrippen und rauher Oberfläche zu schaffen, der eine rasche und großflächige Haftverbindung zwischen der Prothesenoberfläche und dem Knochengewebe ermöglicht. Die erfindungsgemäße Lösung besteht in den Merkmalen des Anspruchs 1, nämlich darin, daß die Keilrippe im Mittel mindestens dreimal so breit wie hoch und die Rauhigkeit auf ihrer Rückenfläche scharfkantig mit einer Rauhtiefe zwischen 0,05 und 0,5 mm ausgebildet ist.

Nach bisherigen Erfahrungen muß man damit rechnen, daß eine derart breite Keilrippe ihre Keilwirkung hauptsächlich nach außen gegen die Knochenrinde entfaltet und dadurch die Gefahr einer sprengung des Knochens heraufbeschworen wird. Diese Gefahr wäre in der Tat vorhanden, wenn die Rückenfläche der Rippe nicht rauh, wäre. Die Rauhigkeit führt dazu, daß die Knochensubstanz, die in unmittelbaren Kontakt mit der Rippenrückenfläche kommt und der Keilwirkung und Relativbewegung ausgesetzt wird, zerrieben und zerkleinert wird und dadurch in einen fließfähigen Zustand gelangt, so daß sie zur Seite weg von der Rippenrückenfläche verdrängt werden und abfließen kann. Dies hat zwei Effekte. Zum einen ist die in Richtung normal zur Rippenrückenfläche erzeugte Kraft vergleichsweise gering, wodurch die möglicherweise schädigende Kraftausübung auf die Knochenrinde begrenzt wird. Zweitens befindet sich die Rippenrückenfläche im Endzustand der Implantation in unmittelbarer Nähe von ungeschädigter Knochensubstanz, in welcher das natürliche Gefäßsystem erhalten geblieben ist, da die zuvor zerriebene Knochensubstanz dank ihrer Fließfähigkeit sich entfernt. Daraus ergibt sich, daß der Vorgang des Einsprossens neuer Knochensubstanz die poröse Rückenfläche der Rippe sehr rasch nach der Implantation erreicht und daher schon nach kurzer Zeit großflächig ein inniger Oberflächenverbund geschaffen wird. Zwar mag es sein, daß auch bei den bisher bekannten Keilrippen mit rauher Oberfläche an deren Querschnittsspitze gleichfalls ein solcher Effekt auftrat. Da er aber auf eine sehr geringe Fläche beschränkt war, wurde er nicht wahrgenommen und fiel er auch nicht positiv ins Gewicht. An den Rippenflanken ist die Situation grundsätzlich anders. Bei einzeln stehenden Rippen ist in Kompressionsrichtung normal zur Flankenoberfläche ein verhältnismäßig weiter Kompressionsraum vorhanden, so daß die Knochensubstanz zwar komprimiert und teilweise gequetscht wird, aber die Pressung nicht so groß wird wie es für eine nennenswerte abrasive Wirkung der Rauhigkeit erforderlich wäre. Die Knochensubstanz bleibt mehr oder weniger an Ort und Stelle. Da die Gefäße darin größtenteils zerstört sind, bildet sie zunächst eine Sperre zwischen dem unverletzen Knochengewebe und der Prothesenoberfläche, die nach der Implantation von frischem Knochengewebe erst durchdrungen werden muß, bevor es in die rauhe Oberfläche der Prothese einsprossen kann und eine formschlüssige Verbindung zustandekommen kann. Auch in den Zwischenräumen zwischen benachbarten Rippen ist die Situation anders als am Rippenrücken, weil dort die Kompression und. Gewebezerstörung besonders ausgeprägt ist, ohne daß sich das zerstörte Gewebe entfernen kann. Auch in diesem Bereich kann also die Verbindung mit frischem Knochengewebe nur verzögert erfolgen.

Die Erfindung schafft somit den Vorteil, daß dank der Rauhheit des großflächigen Rippenrückens und der dadurch bewirkten Nähe zu unverletztem Knochengewebe sehr rasch eine feste Verbindung zwischen Prothesenoberfläche und Knochenoberfläche erfolgen kann.

Vorzugsweise ist die Rippe im gesamten proximalen Abschnitt mindestens dreimal, vorzugsweise viermal so breit wie hoch. Damit die abrasive Wirkung der Rauhigkeit bei der Relativbewegung der Rippenoberfläche gegenüber dem angepressten Knochengewebe stets hinreichend groß ist im Vergleich mit der durch Keilwirkung erzeugten Quetschung, soll der Keilwinkel, das heißt der Winkel zwischen der Fläche des Rippenrückens und der in lateral-medialer-Richtung verlaufenden Mittelebene (LM-Mittelebene) des Schafts, nicht größer als 5°, vorzugsweise nicht größer als 3,5° und weiter vorzugsweise nicht größer als 2,5° sein.

Auch die Breite der Rippe, das heißt ihre Abmessung in LM-Richtung, wächst vorzugsweise von distal nach proximal an, wobei der Winkel zwischen der lateralen Kante und der Längsrichtung des Schafts nicht größer sein sollte als 4°, vorzugsweise 3°. Dasselbe gilt für die laterale Kante.

Im Schaftquerschnitt soll die Rippenrückenfläche etwa parallel zu der LM-Mittelebene verlaufen. Der Winkel zwischen der Rippenrückenfläche und der LM-Mittelebene ist vorzugsweise nicht größer als 15°, wobei die Rippenhöhe zur lateralen Seite hin anwächst. Die mediale Flanke ist zweckmäßigerweise scharfkantig von der Rippenrückenfläche abgesetzt und verläuft im wesentlichen lotrecht zur LM-Ebene. Das selbe gilt zweckmäßigerweise auch für die laterale Kante, obwohl es dort weniger wichtig ist.

Damit die abrasive Wirkung der Rauhigkeit im Bereich des Rippenrückens hinreichend zur Wirkung kommt, liegt der Abstand benachbarter Rauhigkeitsspitzen zweckmäßigerweise in derselben Größenordnung wie die Rauhtiefe, nämlich zwischen 0,05 und 0,5 mm.

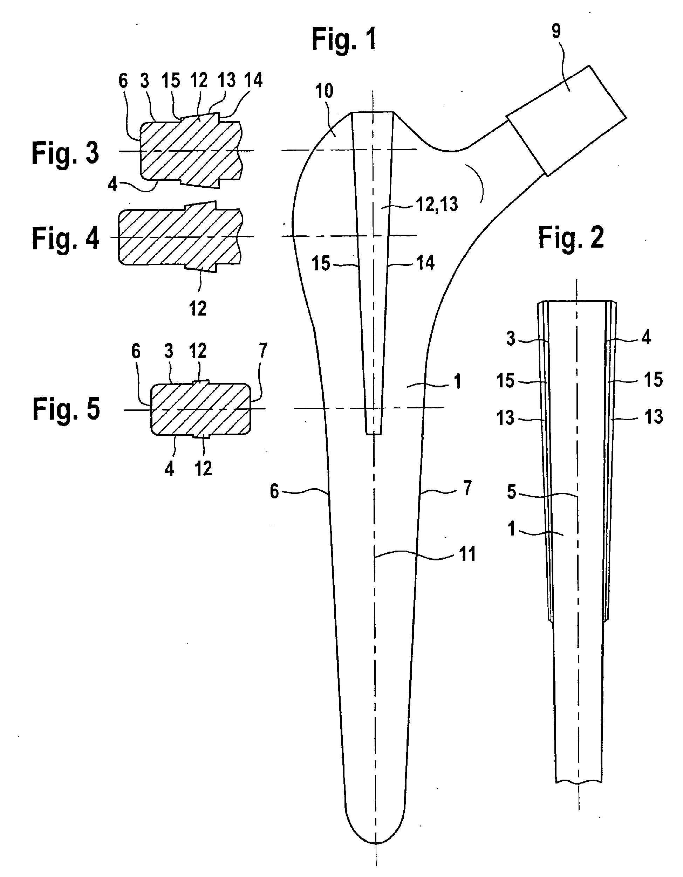

Die Erfindung wird im folgenden näher unter Bezugnahme auf die Zeichnung erläutert, die ein vorteilhaftes Ausführungsbeispiel veranschaulicht. Es zeigen:

| | |
|---|---|
| Figur 1 | eine Seitenansicht |
| Figur 2 | eine Ansicht von lateral und |
| Figur 3 bis 5 | Querschnitte durch den Prothesenschaft in jeweils entsprechender Höhe desselben. |

Es handelt sich um eine Geradschaftprothese, das heißt der Schaft hat eine durchgehend gerade Längsachse und wird in gerader Richtung in das Femur implantiert. Der Schaft 1 umfaßt einen im Querschnitt im wesentlichen rechteckigen Grundkörper mit parallelen dorsalen und ventralen Seiten 3, 4, die sich nach distal keilförmig verjüngen. Sie schließen mit der lateral-medial verlaufenden Mittelebene 5 jeweils einen Winkel von weniger als 2° ein. Die lateralen und medialen Begrenzungsflächen 6, 7 des Schaftgrundkörpers verlaufen gleichfalls nach distal sich keilförmig verjüngend. Am proximalen Ende schließen sich der Prothesenhals mit einem Konus 9 zum Aufsetzen einer Gelenkkugel sowie im Bereich des großen Trochantas ein Lateralflügel 10 an.

Eine nicht dargestellte Raspel zum Formen des Knochenhohlraums, der den Prothesenschaft aufnehmen soll, hat die gleiche Gestalt wie der von den Flächen 3, 4 und 6, 7 begrenzte Grundkörper des Prothesenschafts, wie dies allgemein bekannt ist, um dem Prothesenschaft in der Knochenhöhlung nach der Implantation einen sicheren und festen Sitzt zu verleihen.

Auf die dorsalen und ventralen Flächen 3, 4 des Schaftgrundkörpers sind etwa mittig in Bezug auf die Schaftachse 11 Rippen 12 aufgesetzt, die die Form eines Keils mit geraden Begrenzungsflächen haben. Die Größe dieser Flächen ist die nach dorsal bzw. ventral gewendete Rückenfläche 13. Medial wird die Rippe durch eine etwa lotrecht zur Fläche 3 bzw. 4 verlaufende mediale Stirnfläche begrenzt. Dasselbe gilt für die laterale Stirnfläche 15.

Mit der Mittellinie 11 schließen die Stirnflächen 14, 15 ebenso wie die Begrenzungskanten der Rückenfläche 13 einen Winkel von jeweils etwa 2,5° ein. Der Winkel, den die Rippenrückenflächen 13 mit der LM-Mittelebene des Schafts einschließen, liegt bei 2°.

Die Rippenrückenfläche 13 verläuft etwa parallel zur LM-Mittelebene 5 des Schafts. Die Abweichung beläuft sich im dargestellten Beispiel auf weniger als 10°. An der medialen Kante ist die Rippe etwas höher als an der lateralen Kante, wodurch der makroskopische Formschluß zur Kraftübertragung von der Prothese nach medial auf den Knochen vergrößert wird.

Mindestens die Rückenfläche 13 der Rippe 12 ist durch Sandstrahlen, Plasmabeschichtung, Flammspritzen oder dergleichen mit einer rauhen und gegebenenfalls porösen Oberfläche versehen. Die Rauhigkeitserhebungen sind scharfkantig geformt, so daß sie beim Einschieben des Schafts in den Knochen abrasiv auf die Knochensubstanz wirken. Eine solche Rauhigkeit kann auch an den übrigen Flächen des Prothesenschafts vorgesehen sein, um die innige Verbindung des Knochengewebes mit der Prothesenoberfläche zu ermöglichen. Für die Erfindung kommt es lediglich auf die Rauhigkeit der Rippenrückenfläche 13 an. Während der Schaftgrundkörper in der Knochenhöhlung komplementär vorgeformt ist und darin den gewünschten Presssitz ohne wesentlichen zusätzliche verformung des Knochens findet, fehlt eine solche komplementäre Höhlungsform für die Rippen 12. Beim Eindringen des Schafts in den Knochen verdrängen sie eine ihrem Volumen entsprechende Menge des Knochengewebes. Wäre die Rückenfläche glatt, würde die laminare Struktur des Knochens dabei lediglich zusammengepresst und verdichtet werden, wobei der flüssige Inhalt der lamellaren Zwischenräume entweichen würde. Dank der Abrasivität des Rippenrückens werden im Falle der Erfindung die Knochenlamellen jedoch zerrieben und zerschnitten. Dadurch können sie mit dem flüssigen Zwischenrauminhalt aus dem Bereich zwischen der Rippenrückfläche und dem hinter ihnen liegenden festen und unverletzten Knochengewebe entweichen. Zum einen wird dadurch die Pressung herabgesetzt, die aufgrund der Keilwirkung der Rippen zwischen diesen und dem Knochen entsteht. Zum anderen wird die spongiöse Knochensubstanz, die sich zwischen der Rippenrückenfläche und der harten Knochenrinde befindet, nicht in ihrer Gesamtheit zusammengepresst und geschädigt. Vielmehr bleibt sie bis nahe an die Rippenoberfläche heran gesund und kann daher durch rasch zur Schaftoberfläche vordringendes und in deren Rauhigkeit einsprossendes, frisches Knochengewebe zur raschen Prothesenfestigung beitragen.

## Patentansprüche

1. Satz bestehend aus einer Hüftprothese mit einem in eine Höhlung des Oberschenkelknochens zementfrei einzusetzenden Schaft (1), dessen proximaler Abschnitt aus einem Grundkörper und je einer dorsal und ventral davon vorspringenden Rippe (12) besteht, die parallel zur Schaftrichtung (11) verläuft, im Querschnitt von distal nach proximal zunimmt und eine rauhe Oberfläche aufweist, sowie einen Werkzeug, das zum Formen der mit der Form des Grundkörpers im wesentlichen übereinstimmenden Höhlung ausgebildet ist, **dadurch gekennzeichnet, daß** die Rippe im Mittel mindestens dreimal so breit wie hoch und die Rauhigkeit auf ihrer Rückenfläche (13) scharfkantig mit einer Rauhtiefe zwischen 0,05 und 0,5 mm ausgebildet ist.

2. Hüftprothese nach Anspruch 1, **dadurch gekennzeichnet, daß** sie eine Geradschaftprothese ist.

3. Hüftprothese nach Anspruch 1 oder 2, **dadurch gekennzeichnet, daß** die Rückenfläche (13) der Rippen (12)- im Schaftquerschnitt etwa parallel zur LM-Mittelebene (5) verläuft.

4. Hüftprothese nach Anspruch 3, **dadurch gekennzeichnet, daß** die Höhe der Rippe an einer sie medial begrenzenden Kante (14) größer ist als an ihrer lateralen Begrenzung (15).

5. Hüftprothese nach einem der Ansprüche 1 bis 4, **dadurch gekennzeichnet, daß** eine mediale Stirnfläche (14) der Rippe etwa lotrecht zur Oberfläche (3, 4) des Schaftgrundkörpers verläuft.

6. Hüftprothese nach Anspruch 1, **dadurch gekennzeichnet, daß** der Spitzenabstand der die Rauhigkeit bildenden Erhebungen in derselben Größenordnung wie die Rauhtiefe liegt.

## Claims

1. Set consisting of a hip prosthesis with a shaft (1) which is to be inserted into a cavity in the femur without using cement and whose proximal portion is made up of a base body and of a rib (12) projecting dorsally and a rib (12) projecting ventrally from the base body, said rib extending parallel to the shaft direction (11), increasing in cross section from distal to proximal and having a rough surface, and a tool which is designed to shape the cavity so that it substantially corresponds to the shape of the base body, **characterized in that** the rib is on average at least three times as wide as it is high and the roughness on its rear face (13) has sharp edges with a peak-to-valley height of between 0.05 and 0.5 mm.

2. Hip prosthesis according to Claim 1, **characterized in that** it is a straight shaft prosthesis.

3. Hip prosthesis according to Claim 1 or 2, **characterized in that** the rear face (13) of the ribs (12) extends, in the shaft cross section, approximately parallel to the LM midplane (5).

4. Hip prosthesis according to Claim 3, **characterized in that** the height of the rib is greater at an edge (14) limiting it medially than at its lateral boundary (15).

5. Hip prosthesis according to one of Claims 1 to 4, **characterized in that** a medial end face (14) of the rib extends approximately perpendicular to the surface (3, 4) of the base body of the shaft.

6. Hip prosthesis according to Claim 1, **characterized in that** the distance between the peaks of the elevations forming the roughness is of the same order of magnitude as the peak-to-valley height.

## Revendications

1. Ensemble composé d'une prothèse de hanche comprenant une tige (1) destinée à être ancrée sans ciment dans une cavité du fémur, dont la partie proximale se compose d'un corps de base et d'une nervure (12) saillante chaque fois sur le côté dorsal et ventral de celui-ci, qui est parallèle à la direction (11) de la tige, dont la section transversale augmente de l'extrémité distale à l'extrémité proximale et qui présente une surface rugueuse, ainsi que d'un outil qui est configuré de façon à former la cavité correspondant essentiellement à la forme du corps de base, **caractérisé en ce que** la nervure est en moyenne trois fois plus large que haute et la rugosité sur sa face arrière (13) est à arêtes vives avec une profondeur de rugosité comprise entre 0,05 et 0,5 mm.

2. Prothèse de hanche selon la revendication 1, **caractérisée en ce qu'**elle est une prothèse à tige rectiligne.

3. Prothèse de hanche selon la revendication 1 ou 2, **caractérisée en ce que** la face arrière (13) des nervures (12) dans la section transversale de la tige est sensiblement parallèle au plan central médial longitudinal (5).

4. Prothèse de hanche selon la revendication 3, **caractérisée en ce que** la hauteur de la nervure sur une arête (14) la limitant de façon médiale est plus grande que dans sa limitation latérale (15).

5. Prothèse de hanche selon l'une quelconque des revendications 1 à 4, **caractérisée en ce qu'**une face frontale médiale (14) de la nervure est sensiblement perpendiculaire à la surface (3, 4) du corps de base de la tige.

6. Prothèse de hanche selon la revendication 1, **caractérisée en ce que** la distance des pointes des surélévations formant la rugosité est du même ordre de grandeur que la profondeur de la rugosité.
